# EUROPEAN PATENT SPECIFICATION

(11) **EP 0 667 760 B1**
(45) Date of publication and mention of the grant of the patent: **20.11.1996**
(21) Application number: 93925036.1
(22) Date of filing: 25.10.1993
(51) Int. Cl.: A61F 13/20, A61F 13/26

(54) **TAMPON APPLICATOR**
TAMPONAPPLIKATOR
APPLICATEUR DE TAMPON

(30) Priority: 06.11.1992 US 973072; 06.11.1992 US 973156
(43) Date of publication of application: 23.08.1995
(60) Divisional application: 96201203.5
(73) Proprietor: TAMBRANDS, INC., White Plains, New York 10604 (US)
(72) Inventor: FRAYMAN, Max, Longmeadow, MA 01106 (US); LINDSAY, Richard, J., North Brookfield, MA 01535 (US); SPRAGUE, E., Russell, Palm City, FL 34990 (US); TOMASZEWSKI, Gina, M., Monson, MA 01057 (US); CAVANAUGH, Garrett, Three Rivers, MA 01080 (US)
(74) Representative: Alexander, Thomas Bruce
(86) International application number: US9310207
(87) International publication number: WO9410959

(56) References cited:
- US-A- 4 453 925
- US-A- 4 508 531
- US-A- 5 087 239

## Description

### Background of the Invention

This invention relates to a tampon applicator, formed from a paper laminate.

Tampon applicators comprising a pair of telescopically arranged tubes have long been known in the art. Typically, these applicators comprise a tampon holder tube and a plunger tube, telescopically disposed such that a portion of the plunger tube is within the holder tube.

Some applicators have the tampon exposed at the end intended for vaginal insertion (the expulsion end), while others provide a rounded expulsion end, with the tampon covered by a plurality of segments, referred to in the art as "petals", which open during tampon expulsion. A tampon having the latter construction is disclosed in U.S. Patent No. 5,087,239, the disclosure of which is incorporated herein by reference. In this applicator, a weakening formation, e.g., a groove, is provided at the base of the segments. The weakening formation functions as a hinge, reducing the force required to bend the segments outwardly during expulsion.

However, the force required to bend the segments during expulsion may be undesirably high, even if a groove is provided at the base of the segments. This occurs particularly when a thick, stiff paper laminate, or a laminate including a layer of cellophane or polymeric film, as disclosed in EP-A-0,551,758, is used in a segment construction. The high force required may cause discomfort to the user, or may prevent the tampon from being properly expelled.

### SUMMARY OF THE INVENTION

The invention features an improved tampon applicator for insertion of a tampon into a body cavity, including a paper tampon holder tube having an expulsion end dimensioned for insertion into the body cavity and including a plurality of contiguous petal segments separated by slits, and a plunger, telescopically and slidably mounted in the holder distal to the expulsion end and adapted to expel the tampon from the holder when pushed manually into the holder. As the tampon is expelled, the segments bend open at a hinge. The segments of the improved applicator bend outwardly during expulsion with significantly reduced applied force, improving user comfort.

In one aspect, the tampon holder includes a plurality of small cuts extending circumferentially from the petal slits in the vicinity of the hinge. The cuts may take a variety of forms, so long as they narrow the circumferential extent, i.e., the width, of the petal segments. The cuts reduce the bending moment and rigidity at the hinge, both by shortening the width of the "beam" being bent, and by reducing the curvature of the "beam". In preferred embodiments, the tampon holder is a paper laminate; there is a cut at or near the base of each petal slit; the cuts have a length that effectively narrows the width of the petal segments by anywhere from about 15 to 50 percent.

In preferred embodiments, the tampon holder is a paper laminate; the score lines are combined with the circumferential cuts, both located adjacent the hinge so as to reduce the force required to bend the segments at the hinge; the score lines extend circumferentially, and one or more of the score lines are disposed slightly above (closer to the distal end of the holder tube), and one or more slightly below the weakening formation. Preferably, the depth of the score lines is from about 25 to 80 percent of the total thickness of the paper laminate.

Preferably, one or both of the circumferential cuts and interior score lines is combined with a further weakening formation at the vicinity of the hinge (e.g., a groove or scoring, which may be continuous or discontinuous), as shown in U.S. Patent No. 5,087,239.

For particularly thick or stiff paper laminates, it may be desirable to use both circumferential cuts and interior score lines in combination. For thinner paper laminates, the circumferential cuts or the score lines may be used alone, as the combination of cuts and score lines may in some instances cause the segments to rip off.

Other features and advantages of the invention will be apparent from the following description of preferred embodiments, and from the claims.

### Brief Description of the Drawings

Fig. 1 shows a tampon applicator according to one embodiment of the invention.

Fig. 2 shows a partial side cross-sectional view of the applicator of Fig. 1, enlarged to show detail. Fig. 2a shows a front view of the triangular notches which are removed from the tampon expulsion end to form the petal segments.

Fig. 3 is an enlarged view of the expulsion end of the embodiment shown in Fig. 1.

Figs. 3a-3e show the expulsion ends of tampon applicators according to some of many alternate embodiments of the invention.

### Description of the Preferred Embodiments

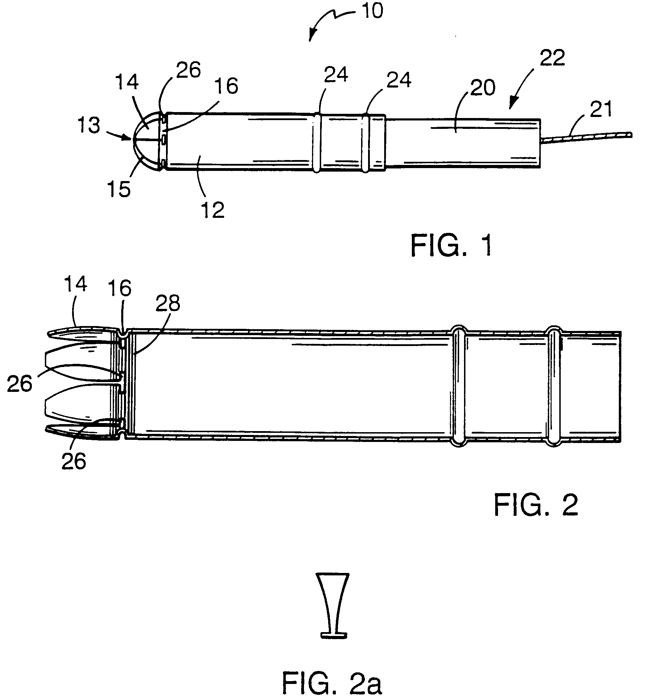

An applicator 10 according to a preferred embodiment of the invention is shown in Figs. 1 and 2. The applicator comprises tubular tampon holder 12, and plunger 20, telescopically and slidably mounted inside of tampon holder 12. A tampon 13 is retained within tampon holder 12, and its expulsion end is surrounded by contiguous petal segments 14, separated by petal slits 15. Petal segments 14 are formed by cutting out triangular notches of the paper laminate (as is known, e.g., in U.S. Patent 5,087,239). This may occur either prior to formation of the tube by stamping, in convolutely wound tampons, or after formation of a spirally wound tube. The segments are then heat-formed into a dome-shape.

In use, as plunger 20 is pressed inwardly, petal segments 14 bend open at a hinge, and the tampon is expelled through the expulsion end.

To reduce the expulsion force required to bend the petal segments open at the hinge, a weakening formation in the form of a groove 16 is embossed into the outer surface of tampon holder 12 at the hinge. Groove 16 extends circumferentially around, at, or near, the base of the segments. The weakening formation may take a variety of forms other than a groove (e.g., it may be a slot or perforation, and it may be continuous or discontinuous). Tampon withdrawal cord 21 extends out of distal end 22 of the applicator. Rings 24 are embossed into tampon holder 12 at its distal end, to provide a gripping surface for the user.

Substantial further reduction in expulsion force is achieved by providing a plurality of small circumferential cuts 26 at the vicinity of the hinge. Each cut extends circumferentially a small distance in each direction from the slit 15. The cuts reduce the bending moment and rigidity at the hinge, both by shortening the width of the "beam" being bent, and by reducing the curvature of the "beam". In a preferred embodiment, each cut is from about 1.02 to 2.29 mm (0.04 to 0.09") long, about 0.25 to 0.76 mm (0.01 to 0.03") high, and has a width approximately equal to that of slits 15.

Further reduction in expulsion force is achieved by providing a zone of indentation, preferably a plurality of score lines 28, disposed on the inside of the tampon holder, and axially overlapping the weakening formation (groove 16) on the exterior. Preferably, groove 16 is aligned in the middle of the zone of indentation (Fig. 2), but this can vary so long as the zone axially overlaps the weakening formation. Score lines 28 are provided across an area extending from slightly above to slightly below groove 16, and are spaced about 0.76 mm (0.030") apart. In alternate embodiments, the zone of indentation could be an embossed band in which the thickness of the holder is reduced; the depth of the embossing could be uniform or varying. If the zone is made up of score lines, there may be more score lines than shown in Fig. 2 (e.g., the score lines may extend further up the segments), or fewer score lines (e.g., only one on either side of the groove), and the score lines may be spaced closer or further apart, preferably from about 0.25 to 1.27 mm (0.010 to 0.050") apart. Preferably, the depth of indentation in the zone is from about 25 to 80 percent of the total thickness of the paper laminate. For the preferred laminate, the score lines are about 0.20 to 0.25 mm (0.008 to 0.010") deep. The score lines may be arranged in many other patterns, e.g., cross-hatching in which score lines intersect, and the scoring can be either continuous or discontinuous provided there is scoring around substantially the entire inner surface of the tampon holder. The score lines may be formed by any suitable process, but are typically formed by a mandrel and rolling die process. The circumferential cuts and score lines may be used in combination (as shown in Fig. 2) or alone, depending on the stiffness of the paper and desired expulsion pressure.

A preferred tampon holder 12 is constructed from a laminate of the following layers: three bleached Kraft outer paper layers and a cellophane layer disposed on the outer tube surface. It is preferred that both the tampon holder and the plunger have this construction, although the holder and the plunger may be made from different materials, e.g., plastic or wax-coated paper, or with ground-wood paper in some of the layers, if desired.

Each of the layers is adhered to adjoining layers by adhesive, preferably a water-based adhesive, to allow the layers to delaminate when the applicator is exposed to water, thereby permitting the applicator to be disposed of by flushing. The water-based adhesive also preserves the biodegradability of the product. Suitable adhesives include, for example, Dextrin™ (vegetable-based adhesive) and polyvinyl acetate, with polyvinyl acetate being preferred for its ability to bond to cellophane under humid storage conditions. Suitable polyvinyl acetate adhesives are commercially available from Findley Adhesives, Wauwatosa, WI.

The cellophane layer may be of any grade of cellophane so long as it has adequate water resistance to maintain its integrity and remain tack-free when used in the invention, i.e. adequate to withstand humid environments for long periods of time, and to withstand insertion into the vagina, without becoming tacky or dissolving. Accordingly, the term does not refer to pure regenerated cellulose which is free from any additives or coatings, for such material has extremely low water resistance. Suitable cellophanes may contain additives or surface coatings which impart water resistance, with surface coatings being preferred. Preferred water resistant coatings are selected from the group consisting of nitrocellulose and polyvinylidene chloride, with nitrocellulose preferred for its biodegradability. A nitrocellulose coated cellophane sheet is commercially available from Flexel, Inc., Atlanta, GA. Water resistance is typically measured in terms of Water Vapor Transmission Rate (WVTR). It is preferred that the cellophane film have a WVTR of less than about 100 g/m²/day. It is preferred that the cellophane layer be thin relative to the paper layer. Preferably the thickness is less than about 0.051 mm (0.0020 inches), more preferably less than 0.023 mm (0.0009 inches).

It is preferred, for aesthetic reasons, that the outer paper layer be a white paper. Although any conventional paper may be used, it is preferred that the paper be selected from the group consisting of bleached Kraft paper and bleached sulfite paper. If no other paper layer is to be utilized, the white paper will be selected to have adequate rigidity to provide structural strength to the finished tube for its use as a tampon applicator. The paper may contain additional optical brighteners, e.g., stilbene derivatives, and the like to enhance the aesthetic appearance of the laminate. The brighteners, and overall whiteness of the paper, work in conjunction with the gloss of the cellophane layer to greatly improve the aesthetic appearance of the resulting applicator. Preferred thicknesses for the outer paper layer are in the range of 0.04 to 0.13 mm (0.0015 to 0.005 inches), more preferably 0.003 inches.

Other polymers may be used in place of cellophane, although with loss of some of the unique advantages of cellophane for this application. A variety of thermoplastic polymers may be used. The polymers should be capable of being formed into a relatively thin sheet, and be themselves water-resistant or have a coating or additive which imparts water-resistance. Preferred polymers include but are not limited to cellophane, polyethylene, polyester, polypropylene, polycaprolactone, and ethylene vinyl acetate.

The tampon holder may have no outer cellophane or polymer layer, provided it has adequate rigidity to withstand the knurling and/or circumferential-cut operations. Suitable laminates which do not have a cellophane or polymer layer should be at least about 0.25 mm (0.010 inch) thick, preferably 0.25 mm to 0.51 mm (0.010 to 0.020 inch) thick.

Any conventional process can be used to form the tube, e.g., spiral or convolute winding of the individual layers, each layer on top of the previous layer about a common central axis. Spiral winding is generally preferred. It is also preferred that the seams formed in each layer during spiral winding be offset from the seams in other layers. These methods are well known to those skilled in the art.

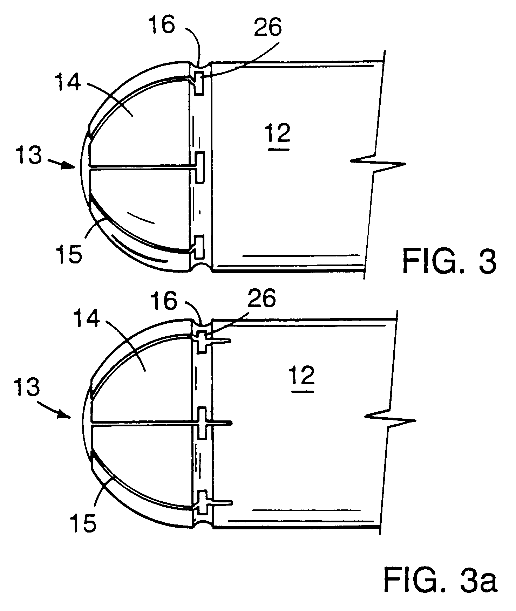

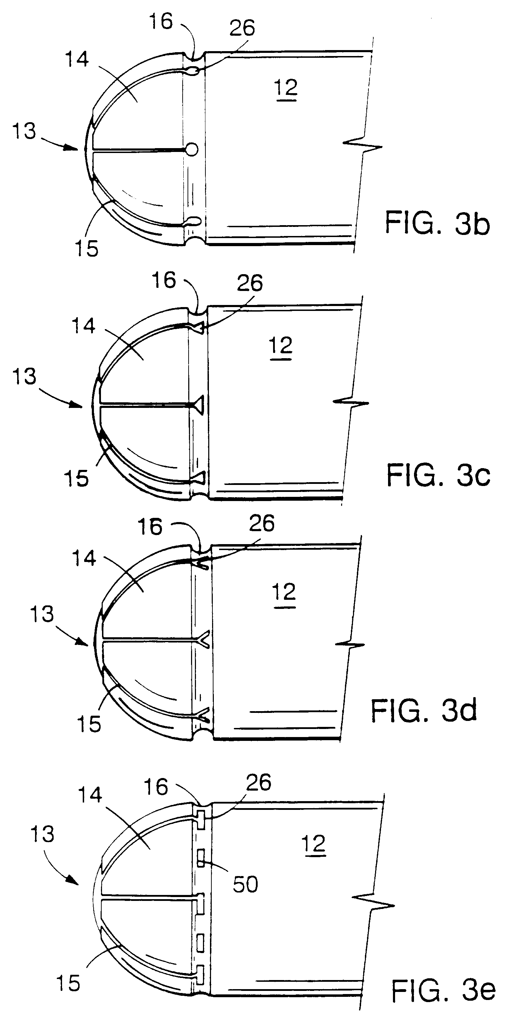

While preferred embodiments have been described above, other variations and modifications are within the scope of the following claims. As examples of the many possible variations, either the circumferential cuts or the score lines could be used by themselves, with or without groove 16 (or some other weakening formation). The slits separating the petal segments can extend beyond the hinge (e.g., as shown in Fig. 3a). The circumferential cuts and score lines need not be precisely aligned with one another, nor with groove 16 (or other weakening formation), although typically expulsion force is minimized if the features are aligned. The circumferential cuts need not extend exactly circumferentially, but could be cut at an angle to the circumferential direction (as shown in Fig. 3d); and other shapes such as circular and triangular cut outs (as shown in Figs. 3b and 3c) could be used, as long as they have some circumferential extent. The cuts need not actually remove material, as shown, but could simply be slits in the paper. The cuts need not be centered on the slits (as shown in Figs. 3 and 3a), but could be offset (as shown in Fig. 3e), and the alignment with slits could vary randomly from slit to slit (Fig. 3e). Additional cuts could be positioned intermediate the slits (as at 50 in Fig. 3e). Although a laminated paper construction has been disclosed, the invention could be applied to nonlaminated paper constructions, and to plastic applicators.

## Claims

1. A tampon applicator for insertion of a tampon into a body cavity, comprising
a tampon holder tube having a longitudinal axis and an expulsion end dimensioned for insertion into the body cavity, the tampon holder tube including a plurality of segments integral with and extending from the expulsion end, each segment having a width and thickness, with slits separating the segments, and
a circumferential hinge at which the segments bend open during expulsion, and
a plunger, telescopically and slidably mounted in the holder and adapted to expel the tampon from the holder when pushed manually into the holder,
characterized in that
the tampon holder includes a plurality of circumferentially-extending cuts, each cut being located adjacent the hinge and extending entirely through the thickness of a segment, and circumferentially from a slit a distance sufficient to narrow the width of the segment, thereby weakening the holder tube at the circumferential hinge and reducing the force required to expel the tampon.

2. The applicator of Claim 1, wherein said tampon holder tube comprises at least one layer of paper.

3. The applicator of Claim 2, wherein said hinge includes a weakening formation.

4. The applicator of Claim 3, wherein said weakening formation comprises a circumferential groove.

5. The applicator of Claim 4, wherein said cuts extend in said circumferential groove.

6. The applicator of Claim 2, wherein said circumferential cuts in said holder tube extend from the ends of the slits separating the segments.

7. The applicator of Claim 2, wherein at each slit there is only one circumferential cut, which extends circumferentially a small distance in one direction from said slit.

8. The applicator of Claim 2, wherein at each slit there are two circumferential cuts, one extending circumferentially a small distance in each circumferential direction.

9. The applicator of Claim 2, wherein said circumferential cuts include cuts having both a circumferential and a longitudinal extent.

10. The applicator of Claim 2, wherein said circumferential cuts reduce said width of the segments from about 15 to 20 percent.

11. The applicator of any of Claims 1 to 10, wherein said tampon holder tube has a dome-shaped expulsion end.

12. The applicator of any of Claims 1 to 11, wherein said tampon holder tube further includes a plurality of circumferentially-extending score lines defining a zone of indentation extending along said longitudinal axis, the score lines being disposed on an inside surface of the tampon holder tube, and the zone axially overlapping the weakening formation.

13. The applicator of Claim 12, wherein said weakening formation comprises a circumferential groove adjacent said hinge and said zone of indentation extends a distance in each direction longitudinally from said groove.

14. The applicator of Claim 13, wherein the tampon holder tube comprises a paper laminate and the depth of the score lines is from about 25 to 80 percent of the total thickness of the paper laminate.

15. The applicator of any preceding claim, wherein said paper is laminated and comprises an outer paper layer and a cellophane layer adhered to said paper layer on its outer surface, said cellophane layer having adequate water resistance to maintain its integrity during insertion into the body cavity.

## Patentansprüche

1. Tampon-Applikator zur Einführung eines Tampons in einen Körperhohlraum, umfassend:
eine Tamponhalterhülse mit einer Längsachse und einem Ausstoßende, das zur Einführung in den Körperhohlraum dimensioniert ist, wobei die Tamponhalterhülse eine Mehrzahl von Segmenten umfaßt, die mit dem Ausstoßende integral ausgebildet sind und sich von diesem erstrecken, wobei jedes Segment eine Breite und eine Dicke aufweist und Schlitze die Segmente trennen, und
ein Umfangsscharnier, an welchem sich die Segmente während der Ausstoßung aufbiegen, und
ein Kolben, der in dem Halter teleskopartig und verschiebbar angebracht ist und ausgeführt ist, den Tampon aus dem Halter auszustoßen, wenn der Kolben manuell in den Halter gedrückt wird,
dadurch gekennzeichnet,
daß der Tamponhalter eine Mehrzahl von sich in Umfangsrichtung erstreckenden Einschnitten umfaßt, wobei jeder Einschnitt benachbart zu dem Scharnier angeordnet ist und sich vollständig durch die Dicke eines Segments erstreckt sowie von einem Schlitz eine Strecke in Umfangsrichtung erstreckt, die ausreichend ist, um die Breite der Segmente zu verringern, wodurch die Halterhülse an dem Umfangsscharnier geschwächt und die zum Ausstoßen des Tampons benötigte Kraft reduziert wird.

2. Applikator nach Anspruch 1, bei welchem die Tamponhalterhülse wenigstens eine Papierschicht umfaßt.

3. Applikator nach Anspruch 2, bei welchem das Scharnier eine Schwächungsausbildung umfaßt.

4. Applikator nach Anspruch 3, bei welchem die Schwächungsausbildung eine Umfangsnut umfaßt.

5. Applikator nach Anspruch 4, bei welchem sich die Einschnitte in der Umfangsnut erstrecken.

6. Applikator nach Anspruch 2, bei welchem sich die Umfangseinschnitte in der Halterhülse von den Enden der Schlitze erstrecken, die die Segmente trennen.

7. Applikator nach Anspruch 2, bei welchem an jedem Schlitz lediglich ein Umfangseinschnitt ist, der sich in Umfangsrichtung von dem Schlitz eine kleine Strecke in eine Richtung erstreckt.

8. Applikator nach Anspruch 2, bei welchem an jedem Schlitz zwei Umfangseinschnitte sind, wobei sich in beide Umfangsrichtungen jeweils ein Schlitz eine kleine Strecke in Umfangsrichtung erstreckt.

9. Applikator nach Anspruch 2, bei welchem die Umfangseinschnitte Einschnitte umfassen, die sowohl eine Umfangs- als auch eine Längsausdehnung aufweisen.

10. Applikator nach Anspruch 2, bei welchem die Umfangseinschnitte die Breite der Segmente um etwa 15 bis 20 Prozent reduzieren.

11. Applikator nach einem der Ansprüche 1 bis 10, bei welchem die Tamponhalterhülse ein kuppelförmiges Ausstoßende aufweist.

12. Applikator nach einem der Ansprüche 1 bis 11, bei welchem die Tamponhalterhülse ferner umfaßt: eine Mehrzahl von in Umfangsrichtung sich erstreckenden Rillenlinien, die eine Vertiefungszone festlegen, welche sich längs der Längsachse erstreckt, wobei die Rillenlinien an einer Innenseitenfläche der Tamponhalterhülse angeordnet sind und die Zone die Schwächungsausbildung axial überlappt.

13. Applikator nach Anspruch 12, bei welchem die Schwächungsausbildung eine Umfangsnut benachbart zu dem Scharnier umfaßt und wobei sich die Vertiefungszone eine Strecke in jede Richtung längs von dieser Nut erstreckt.

14. Applikator nach Anspruch 13, bei welchem die Tamponhalterhülse einen Papierschichtstoff umfaßt und die Tiefe der Rillenlinien etwa 25 bis 80 Prozent der Gesamtdicke des Papierschichtstoffs ausmacht.

15. Applikator nach einem der vorhergehenden Ansprüche, bei welchem das Papier geschichtet ist und eine äußere Papierschicht und eine Cellophanschicht umfaßt, die an der Papierschicht an ihrer äußeren Fläche haftet, wobei die Cellophanschicht eine geeignete Wasserbeständigkeit aufweist, um ihre Unversehrtheit während der Einführung in den Körperhohlraum zu behalten.

## Revendications

1. Un applicateur de tampon, pour insertion d'un tampon dans une cavité corporelle, comprenant :
un tube support de tampon, ayant un axe longitudinal et une extrémité d'expulsion dimensionnée pour produire une insertion dans la cavité corporelle, le tube support de tampon comprenant une pluralité de segments réalisés d'un seul tenant avec, et s'étendant depuis, l'extrémité d'expulsion, chaque segment ayant une largeur et une épaisseur, avec des fentes séparant les segments, et
une charnière circonférentielle sur laquelle les segments fléchissent en s'ouvrant lors de l'expulsion, et
un plongeur, monté de façon télescopique et coulissante dans le support et adapté pour expulser le tampon depuis le support lorsqu'il est poussé manuellement dans le support,
caractérisé en ce que
le support de tampon comprend une pluralité de découpures s'étendant circonférentiellement, chaque découpure étant placée de façon adjacente à la charnière, traverant entièrement l'épaisseur d'un segment, et s'étendant circonférentiellement à partir d'une fente sur une distance suffisante pour rétrécir la largeur du segment, de manière à affaiblir le tube support à la charnière circonférentielle et à réduire la force nécessaire pour l'expulsion du tampon.

2. L'applicateur selon la revendication 1, dans lequel ledit tube support de tampon comprend au moins une couche de papier.

3. L'applicateur selon la revendication 2, dans lequel ladite charnière comprend une formation d'affaiblissement.

4. L'applicateur selon la revendication 3, dans lequel ladite formation d'affaiblissement comprend une gorge circonférentielle.

5. L'applicateur selon la revendication 4, dans lequel lesdites découpures s'étendent dans ladite gorge circonférentielle.

6. L'applicateur selon la revendication 2, dans lequel lesdites découpures circonférentielles ménagées dans ledit tube support s'étendent depuis les extrémités des fentes séparant les segments.

7. L'applicateur selon la revendication 2, dans lequel à chaque fente se trouve une seule découpure circonférentielle, s'étendant circonférentiellement sur une petite distance dans une direction à partir de ladite fente.

8. L'applicateur selon la revendication 2, dans lequel à chaque fente se trouvent deux découpures circonférentielles, s'étendant chacune circonférentiellement sur une petite distance dans chaque direction circonférentielle.

9. L'applicateur selon la revendication 2, dans lequel lesdites découpures circonférentielles comprennent des découpures ayant une étendue à la fois circonférentielle et longitudinale.

10. L'applicateur selon la revendication 2, dans lequel lesdites découpures circonférentielles réduisent ladite largeur des segments d'environ 15 à 20 pour cent.

11. L'applicateur selon l'une quelconque des revendications 1 à 10, dans lequel ledit tube support de tampon a une extrémité d'expulsion en forme de dôme.

12. L'applicateur selon l'une quelconque des revendications 1 à 11, dans lequel ledit tube support de tampon comprend en outre une pluralité de lignes d'entaillage s'étendant circonférentiellement, définissant une zone d'indentation s'étendant suivant ledit axe longitudinal, les lignes d'entaillage étant disposées sur une surface intérieure du tube support de tampon, et la zone chevauchant axialement la formation d'affaiblissement.

13. L'applicateur selon la revendication 12, dans lequel ladite formation d'affaiblissement comprend une gorge circonférentielle placée en position adjacente à ladite charnière et ladite zone d'indentation s'étend sur une certaine distance dans chaque direction, longitudinalement depuis ladite gorge.

14. L'applicateur selon la revendication 13, dans lequel le tube support de tampon comprend un laminé en papier et la profondeur des lignes d'entaillage est d'environ 25 à 80 pour cent de l'épaisseur totale du laminé en papier.

15. L'applicateur selon l'une quelconque des revendications précédentes, dans lequel ledit papier est laminé et comprend une couche de papier extérieure et une couche en Cellophane en adhérence sur ladite couche de papier sur sa surface extérieure, ladite couche de Cellophane ayant une résistance à l'eau appropriée pour conserver son intégrité lors de l'insertion dans la cavité corporelle.
